(19) Europäisches Patentamt

European Patent Office

Office européen des brevets

(11) Veröffentlichungsnummer: **0 356 382 B1**

(12) **EUROPÄISCHE PATENTSCHRIFT**

(45) Veröffentlichungstag der Patentschrift: **16.02.94**

(21) Anmeldenummer: **89810566.3**

(22) Anmeldetag: **25.07.89**

(51) Int. Cl.⁵: **A61K 9/70, A61K 47/22**

(54) **Mehrschichtiges Pflaster.**

(30) Priorität: **02.08.88 CH 2920/88**

(43) Veröffentlichungstag der Anmeldung:
**28.02.90 Patentblatt 90/09**

(45) Bekanntmachung des Hinweises auf die
Patenterteilung:
**16.02.94 Patentblatt 94/07**

(84) Benannte Vertragsstaaten:
**AT BE CH DE ES FR GB GR IT LI LU NL SE**

(56) Entgegenhaltungen:
**EP-A- 0 285 563**
**EP-A- 0 341 202**
**FR-A- 2 527 450**

(73) Patentinhaber: **CIBA-GEIGY AG**
**Klybeckstrasse 141**
**CH-4002 Basel(CH)**

(72) Erfinder: **Sinnreich, Joel, Dr.**
**Hohe Winde Strasse 98**
**CH-4059 Basel(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft ein mehrschichtiges Pflaster für die transdermale Verabreichung von hautpermeationsfähigen Wirkstoffen sowie ein Verfahren zur Herstellung dieses Pflasters.

Die topische Applikation von systemischen Wirkstoffen mit Cremen, Salben, Pasten, Lotionen etc. kann im Gegensatz zu anderen parenteralen Verabreichungsformen, wie intravenös, von fast allen Patienten selbst vorgenommen werden und ist auch schmerzfrei. Gegenüber der oralen Verabreichung mit Tabletten oder Kapseln bietet sich die topische Applikation als Alternative an, wenn der Abbau des Wirkstoffs in der Leberpassage zu hohen Dosierungen mit erhöhtem Risiko von Nebenwirkungen zwingt.

Es ist bekannt, dass man die Permeabilität von systemischen Wirkstoffen mit bestimmten, deren Hautpenetration fördernden Mitteln ("Hautpenetriermittel oder Penetriermittel, Flux Enhancer"), z.B. mit Dimethylsulfoxid, Dimethylformamid oder Methyl-n-dodecylsulfoxid, siehe U.S. Patentschrift 3,257,864, oder 1-n-Dodecylazacyclohexan-2-on, siehe U.S. Patentschrift 4,316,893, wesentlich steigern kann. Diese Hilfsstoffe sind auch als besonders starke, die Permeabilität von vielen systemischen Wirkstoffen steigernde Mittel bekannt. Die Eignung von Eucalyptol und Gemischen von Eucalyptol mit anderen Hautpenetriermitteln, insbesondere im Mischungsverhältnis von 1:1 mit N-Methyl-2-pyrrolidon, zur Steigerung der Permeabilität von systemischen Wirkstoffen in topischen Präparaten wie Crenen, Salben, Pasten, Lotionen etc. ist in der Europäischen Patentanmeldung 69 385 beschrieben.

Die genannten topischen Arzneiformen sind allerdings nur kurze Zeit applizierbar, müssen oft erneuert werden und erlauben generell wegen ihrer ungleichmässigen Verteilung auf der Haut nur eine ungenaue Dosierung des Wirkstoffs. Als Alternative zur Applikation mit solchen Arzneiformen bietet sich die Applikation von systemischen Wirkstoffen mit transdermalen therapeutischen Systemen vor allem dann an, wenn eine kontinuierliche Freisetzung der Wirkstoffkomponente mit kontrollierter Dosierung über einen längeren Zeitraum beabsichtigt ist.

Zur Illustration des Standes der Technik wird die publizierte Französische Patentanmeldung 2 527 450 genannt, worin Reservoirschichten bestehend aus natürlichem oder synthetischem Kautschuk beschrieben sind.

Wegen der geringen Durchlässigkeit der Haut, insbesondere der äusseren Hornhautschicht, müssen die mit solchen therapeutischen Systemen transdermal zu applizierenden systemischen Wirkstoffe allerdings folgendes Anforderungsprofil erfüllen:

1. Sie müssen trotz der Sperrfunktion der äusseren Hornschicht ausreichende Hautpermeabilität besitzen, um in den Blutkreislauf zu gelangen;
2. Sie müssen gute Hautverträglichkeit aufweisen;
3. Sie müssen ausserdem für eine prophylaktische oder therapeutische Daueranwendung oder eine Substitutionstherapie geeignet sein (siehe auch H. Asche, Pharma International 4 (P), 1984, S. 162).

Dieses strenge Anforderungsprofil begrenzt die Auswahl der zur Verfügung stehenden transdermal applizierbaren Wirkstoffe, 50 dass entsprechende therapeutische Systeme nur mit wenigen Wirkstoffen bisher Verwendung in der Therapie gefunden haben, z.B. mit den Wirkstoffen Scopolamin, Nitroglycerin, Estradiol oder Clonidin.

Die Verwendung der genannten Hautpenetriermittel in transdermalen therapeutischen Systemen ist ebenfalls problematisch. Aufgrund ihres hohen Lösungsvermögens für polymeres organisches Material können diese die Formbeständigkeit der Bestandteile des Systems nachteilig verändern. In den technisch einfach herzustellenden Matrixsystemen löst sich die Matrixschicht von der Deckschicht oder einer gegebenenfalls vorhandenen Klebschicht ab. Mitunter wird die Klebewirkung der Matrixschicht unbeabsichtigt so stark, dass sich das Pflaster nur mit erhöhtem Kraftaufwand wieder entfernen lässt.

Der vorliegenden Erfindung liegt die Aufgabe zugrunde, eine Applikationsform für systemische Wirkstoffe in Form eines transdermalen therapeutischen Matrixsystems mit verbesserten Stabilitäts- und Abgabeeigenschaften herzustellen. Diese Aufgabe wird durch die vorliegende Erfindung gelöst, welche ein besonders vorteilhaftes transdermales therapeutisches System in Form eines mehrschichtigen Pflasters mit einer Matrixschicht aus gegebenenfalls vernetzten Styrol-Blockmischpolymerisaten vermischt mit Alkan- oder Alkadienhomopolymeren und besonders günstigen Abgabeeigenschaften, Formbeständigkeit und Klebewirkung betrifft.

Das mehrschichtige Pflaster hat folgende Bestandteile:

a) eine für die Bestandteile der Reservoirschicht
b) undurchlässige Deckschicht;

b) eine Wirkstoff-abgabefähige Reservoirschicht, welche aus gegebenenfalls vernetztem Blockmischpolymerisat auf der Basis von Styrol, Alkadienen und gegebenenfalls Alkenen vermischt mit klebefähigen Polymeren besteht und gegebenenfalls mit einer zusätzlichen klebefähigen Membranschicht versehen ist, und mindestens einen hautpermeationsfähigen Wirkstoff, eine Kombination aus Eucalyptol mit einem Gehalt von mindestens 70 % 1,8-Cineol und N-Methyl-2-pyrrolidon als ein die Hauptpermeabilität von Wirkstoffen förderndes Mittel und gegebenenfalls weitere pharmazeutische Hilfsstoffe enthält, und

c) eine von der Reservoirschicht abziehbare Schutzfolie.

Die weiter vorn und im folgenden verwendeten Begriffe und Definitionen haben im Rahmen der Beschreibung der vorliegenden Erfindung vorzugsweise die folgenden Bedeutungen:

Die Deckschicht a) besteht aus einem polymeren Material oder einer Kombination von polymeren Materialien, die für die Bestandteile der Reservoirschicht b), insbesondere flüssige Bestandteile der Formulierung, undurchlässig sein müssen. Sie dient als äussere Schutzschicht des Systems. Zur Herstellung dieser Deckschicht können gegebenenfalls mit Metallfolie wie Aluminiumfolie beschichtete Hoch- oder Niederdruckpolymere wie Polyäthylen, Polypropylen, Polyvinylchlorid, Polyäthylenterephthalat, Celluloseacetat, Vinylacetat-Vinylchlorid-Copolymere, Polyacrylate, Polyester oder Aethylen-Vinylacetat-Copolymere verwendet werden. Bevorzugt ist eine undurchlässige, flexible Deckschicht, welche sich der Ausformung der betreffenden Körperpartie anpasst, worauf das Pflaster angebracht ist.

Die Reservoirschicht b) befindet sich zwischen der Deckschicht a) und der Schutzfolie c) und besteht aus einer Mischung von hautverträglichen, gegebenenfalls vernetzten Blockmischpolymerisaten auf der Basis von Styrol, Alkadienen und gegebenenfalls Alkenen vermischt mit klebefähigen Polymeren. Die Reservoirschicht b) enthält mindestens einen hautpermeationsfähigen Wirkstoff eine Kombinationen aus Eucalyptol mit einem Gehalt von mindestens 70% 1,8-Cineol und N-Methyl-2-pyrrolidon sowie gegebenenfalls weitere pharmazeutische Hilfsstoffe. Die Reservoirschicht b) kann zusätzlich eine permeable und klebefähige Membran zur Steuerung der Abgabemenge pro Zeiteinheit des zu applizierenden Wirkstoffs aus dem System auf die Haut und/oder zur Verbesserung der Hafteigenschaften eine zusätzliche Klebschicht enthalten.

Blockmischpolymerisate auf der Basis von Styrol, Alkadienen und gegebenenfalls Alkenen sind bekannt und gehören zur Gruppe der Synthesekautschuke mit unregelmässiger Anordnung der Doppelbindungen in der Kohlenstoffkette, welche durch Copolymerisation von Alkadienen mit Styrol oder durch Polymerisation von Alkadienen und Alkenen mit Styrol gebildet werden. Die Copolymerisation kann insbesondere zwischen $C_4$-$C_6$-Alkadienen, z.B. Butadien oder Isopren, und Styrol oder zwischen den genannten $C_4$-$C_6$-Alkadienen und $C_2$-$C_4$-Alkenen, z.B. Aethylen oder Propylen, und Styrol erfolgen. Bevorzugt sind Blockmischpolymerisate mit einem Alkadien-Monomerengehalt oder einem Alkadien-Alken-Monomerengehalt von mindestens 40%, vorzugsweise 50 %. Besonders bevorzugt sind die Blockmischpolymerisate auf der Basis von Styrol, Alkadienen und gegebenenfalls Alkenen, welche als Synthesekautschuke von der Firma Shell unter den Warenzeichen CARIFLEX und KRATON vertrieben werden.

Die genannten Blockmischpolymerisate können durch Zusatz von üblichen Vernetzungsmitteln wie Schwefel oder Schwefel-abgabefähigen Additiven wie Dimorpholyldisulfid, 2-Morpholinodithiobenzothiazol, Caprolactamdisulfid oder Dipentamethylenthiuramtetrasulfid (Vulkanisiermitteln), gegebenenfalls unter Zusatz von Vulkanisationsbeschleunigern, insbesondere vom Typ Sulfenamide, Triazine, Guanidine, Thiurame, Dithiocarbamate, Xanthogenate, Aldehydamin-Kondensationsprodukten, Aminen oder Thioharnstoffen unter Anwendung der üblichen Vulkanisierverfahren, vorzugsweise bei Temperaturen unterhalb 100°C, vernetzt sein. Statt mit Vulkanisiermitteln lassen sich die Blockmischpolymerisate vorzugsweise durch Einwirkung von energiereicher Strahlung ganz oder teilweise, oberflächlich oder durchgehend vernetzen, gegebenenfalls unter Zusatz von Photosensibilisatoren, z.B. mit Gammastrahlung, z.B. aus $^{60}$Co-Quellen, Elektronenstrahlung oder ultravioletter Strahlung in einem Wellenlängenbereich von ca. 200 bis 400 nm, welche man in Quecksilberdampflampen mit Hochdruck- oder Niederdruckentladung erzeugt.

Besonders bevorzugt sind Butadien-Styrol-Blockcopolymere und AethylenButadien-Styrol-Blockpolymere, insbesondere vom Typ KRATON, z.B. KRATON GX 1657, oder CARIFLEX, z.B. CARIFLEX TR 107, wobei letztere durch Einwirken von energiereicher Strahlung, z.B. ultravioletter Strahlung ganz oder teilweise vernetzt sind.

Klebefähige Polymere sind beigemischt, welche sowohl für den Wirkstoff als auch für das Penetriermittel permeabel sind und neben Hautverträglichkeit ausreichende Klebefähigkeit besitzen.

Solche klebefähigen Polymere sind z.B. Siliconkautschuk (Silicone), z.B. lineare Polysiloxane, worin die Siliciumatome in der Siloxankette durch zwei gleiche oder verschiedene Alkyl-, z.B. Methyl- oder Aethyl-, Aryl-, z.B. Phenyl-, Alkenyl-, z.B. Vinyl- oder Allyl-, Alkylaryl-, z.B. Tolyl- oder Xylyl-, oder Aralkyl-, z.B. Benzylreste, und die terminalen Siliciumatome durch drei der genannten organischen Reste substituiert sind. Die Herstellung dieser Silicone ist in den U.S. Patentschriften 2,541,137, 2,723,966, 2,863,846, 2,890,188, 2,927,907, 3,002,951 und 3,035,016 beschrieben, wobei bei Raumtemperatur vulkanisierbare Silicone bevorzugt sind.

Klebefähige Polymere sind ferner hydrophile Polymerisate von Monoestern ungesättigter Carbonsäuren wie Acrylsäure oder Methacrylsäure, z.B. deren Polyhydroxyäthylacrylate oder Polyhydroxyäthylmethacrylate, deren Herstellung in den U.S. Patentschriften 2,976,576 und 3,220,960 beschrieben ist, sowie Copolymere aus wasserlöslichen aliphatischen oder zyklischen Vinylamiden, z.B. Poly-N-vinyl-methylacetamid, -äthylacetamid, -methylpropionamid, -äthylpropionamid, -methylisobutyramid, -2-pyrrolidon, -2-piperidon, -epsilon-caprolactam, -5-methyl-2-pyrrolidon oder -3-methyl-2-pyrrolidon, insbesondere Poly-N-vinylpyrrolidon mit einer mittleren Molmasse von ca. 10 000 - 360 000, mit wasserlöslichem Polyvinylacetat oder Polyvinylalkohol mit unterschiedlichem Acetatgehalt, z.B. Polyvinylacetat mit einer Molmasse von ca. 5 000 bis 400 000 oder Polyvinylalkohol mit einem Hydrolysegrad von ca. 85-98 % und einem Polymerisationsgrad von ca. 500 - 2 500.

Bevorzugt sind Alken- oder Alkadienhomopolymere beigemischt, welche die Elastizität und Klebfähigkeit der Reservoirschicht b) erhöhen. Solche Homopolymere sind z.B. Hochdruck- oder Niederdruckpolyäthylen, Polypropylen, Polybutadien, z.B. Butadien-Natrium ("Zahlenbuna" vom Typ 32 oder 85) oder cis-1,4-Polybutadien, Polyisopren oder insbesondere Polyisobutylen mit einem durchschnittlichen Molekulargewichtsbereich von ca. $1,0 \times 10^3$ bis $5,0 \times 10^4$, z.B. Polyisobutylen der Bezeichnung OPPANOL (BASF), z.B. OPPANOL B-10. Zur Verbesserung der Haftfähigkeit können noch die in der Fachliteratur bekannten Harze verwendet werden, z.B. Kolofon.

Die Reservoirschicht b), welche den permeationsfähigen Wirkstoff, z.B. Progesteron, und das Hautpenetriermittel Eucalyptol kombiniert mit N-Methyl-2-pyrrolidon enthält, ist auf der Haut ausreichend klebefähig, so dass das Pflaster mindestens einen Tag lang dort befestigt bleibt und sich danach ohne erhöhten Kraftaufwand entfernen lässt. Ausserdem zeichnet sich das Pflaster durch gute Abgabeleistungen des zu applizierenden Wirkstoffs aus, so dass die Abgabe von therapeutisch wirksamen Mengen des transdermal zu applizierenden Wirkstoffs gewährleistet ist. Durch Oberflächenvernetzung, z.B. kurzem Bestrahlen mit energiereichem Licht, kann die Reservoirschicht b), die Eigenschaft einer permeablen Membran annehmen, welche die Durchlässigkeit für den Wirkstoff und das Penetriermittel steuert.

Die Reservoirschicht b) kann ausserdem mit einer zusätzlichen permeablen Membran versehen sein, welche die erforderliche Durchlässigkeit für den Wirkstoff und das Penetriermittel besitzt. Diese Schicht steuert zusätzlich die Abgabegeschwindigkeit des Penetriermittels und gegebenenfalls der Wirkstoffkombination aus dem System auf die Haut und wird auch als Kontroll- oder Steuermembran bezeichnet.

Die für die Herstellung der permeablen Membran verwendbaren Materialien sind an sich bekannt. Solche Membranmaterialien können homogen (Diffusionsmembranen), oder makrostrukturiert (poröse Membranen) sein. Letztere können als schwammförmiges Gebilde mit einer löchrigen Gerüststruktur aus polymerem Material aufgefasst werden, worin untereinander verbundene Zwischenräume und Poren dispergiert sind. Membranmaterialien, welche die Abgabegeschwindigkeit steuern, können aus isotropem Material mit homogener Struktur oder aus anisotropem Material mit nicht-homogener Struktur bestehen. Solche Materialien sind kommerziell erhältlich und können auf verschiedene Weise hergestellt werden, z.B. wie von R.E. Kesting, Synthetic Polymer Membranes, McGraw Hill, Chapters 4 und 5, 1971, J.D. Ferry, Ultrafiltration Membranes, Chemical Review, Vol. 18, Seite 373, 1984, beschrieben.

Membranmaterialien mit 5 bis 95 Vol% Hohlräumen und einer effektiven Porengrösse von ca. $1,0 \times 10^{-9}$ m bis $1,0 \times 10^{-4}$ m sind besonders geeignet. Vor allem eignen sich Membranmaterialien mit Porengrössen kleiner als ca. $5,0 \times 10^{-9}$ m und molekularer Diffusion. Für optimale Resultate wird auf den Stand der Technik und die bekannten Ausführungsformen mit bekannten Membranmaterialien und bekannter Formgebung verwiesen, welche eine optimale Abgabegeschwindigkeit des Wirkstoffs oder der Wirkstoffkombination gewährleisten. Insbesondere muss das Membranmaterial gegenüber dem Wirkstoff oder der Wirkstoffkombination und dem verwendeten Penetriermittel chemisch resistent sein.

Im folgenden ist eine Aufzählung von geeigneten Membranmaterialien angegeben, welche als nicht erschöpfend aufzufassen ist:

Polycarbonate, z.B. lineare Polyester von Kohlensäurederivaten, welche Carbonatgruppen in der Polymerkette enthalten, und z.B. durch Umsetzung von Dihydroxyaromaten mit Phosgen herstellbar sind. Solche Materialien sind unter dem Warenzeichen Lexan® von General Electric erhältlich;

Polyvinylchloride, z.B. das PVC, welches unter dem Warenzeichen Geon® 121 der Firma Goodrich erhältlich ist;

Polyamide vom Typ Polyhexamethylenadipamid oder solche Polyamide, welche unter dem generischen Namen "Nylon" bekannt sind. Ein besonders geeignetes Material wird unter dem Warenzeichen Nomex® von DuPont vertrieben;

Acrylsäurecopolymere, z.B. solche, welche unter den Handelsnamen Dynel® verkauft werden und zu ca. 60 % aus Polyvinylchlorid und zu 40 % aus copolymerisiertem Acrylnitril bestehen, sowie Styrol-Acrylsäurecopolymere und ähnliche;

Polysulfone mit Diphenylsulfon-Gruppen in der linearen Kette. Solche Polymere werden von Union Carbide unter der Bezeichnung P-1700 vertrieben; Halogenierte Polymere wie Polyvinylidenfluoride, welche z.B. unter dem Warenzeichen Kynar® von Pennwalt vertrieben werden; Polyvinylfluoride, welche unter dem Warenzeichen Tedlar® bei DuPont erhältlich sind, sowie Polyfluorohalocarbon erhältlich unter dem Warenzeichen Aclar® bei Allied Chemical;
Polychloräther, welche unter dem Warenzeichen Penton® von Hercules vertrieben werden, sowie andere ähnliche thermoplastische Polymere;
Acetalpolymere wie die Polyformaldehyd-Polymere, welche von DuPont unter dem Warenzeichen Delrin® vertrieben werden u.ä.;
Acrylsäureresinate wie Polymethylmethacrylat, Poly-n-butyl-methacrylat u.ä.;
Polyäthylen und Copolymere des Aethylens z.B. mit Vinylacetat oder Acrylaten.

Bei Verwendung einer permeablen Membran sind mehrere Anordnungen möglich: Der Wirkstoff und das Penetriermittel befinden sich in der aus Blockmischpolymerisat und Alken- oder Alkadienhomopolymerisat gebildeten Matrix zwischen der Deckschicht a) und der permeablen Membran.

Man kann auch gemäss der in der Deutschen Offenlegungsschrift 3,205,258 beschriebenen Ausführungsform das von der Deckschicht a) und der permeablen Membran gebildete Volumen nur mit dem Penetriermittel, z.B. Eucalyptol, und dem Gemisch aus Blockmischpolymerisat und Alkan- oder Alkenhomopolymerisat füllen und auf der anderen Seite der Membran den Wirkstoff oder eine Wirkstoffkombination auftragen. Die Membran steuert dann nur die Diffusionsgeschwindigkeit des Penetriermittels. Der Wirkstoff oder die Wirkstoffkombination kann auch in einer getrennten Schicht zwischen Membran und einer zusätzlichen Klebematerial und gegebenenfalls oder ausschliesslich in dem zusätzlichen Klebematerial vorhanden sein, welches sich auf der Membranschicht befinden kann.

Zur Erhöhung der Klebefähigkeit eignen sich die in der Dermatologie verwendbaren Klebematerialien. Geeignete Klebematerialien sind beispielsweise klebrige Formulierungen von Acrylsäure- oder Methacrylsäureharzen, z.B. Polymere von Acrylsäure oder Methacrylsäure verestert mit Alkoholen wie n-Butanol, n-Pentanol, Isopentanol, 2-Methylbutanol, 1-Methylbutanol, 1-, 2- oder 3-Methylpentanol, 2-Aethylbutanol, Isooctanol, n-Decanol oder n-Dodecanol, oder Copolymerisate dieser Acrylsäure- oder Methacrylsäureester mit Aethylengruppen-haltigen Monomeren wie Acrylsäure selbst, Methacrylsäure, Acrylamid, Methacrylamid, N-Alkoxymethacrylamid, N-Alkoxymethylmethacrylamid, N-tert-Butylamid, Itaconsäure, Vinylacetat, N-verzweigtes Alkylmaleinsäureamid, worin die verzweigte Alkylgruppe 10-24 C-Atome hat, Glycoldiacrylate oder Mischungen davon, natürlicher oder synthetischer Kautschuk wie Styrolbutadien, Butyläther, Neopren, Polyisobutylen, Polybutadien und Polyisopren, Polyvinylacetat, Harnstoff-Formaldehyd-Resinate, Resorcin-Formaldehyd-Resinate, Cellulosederivate wie Aethylcellulose, Methylcellulose, Nitrocellulose, Celluloseacetatbutyrat und Carboxymethylcellulose sowie natürliche Kleber wie Guar, Acacia, Pektin, Stärke, Dextrin, Albumin, Gelatine, Casein etc. Die genannten Klebstoffe können noch mit Verdickern und Stabilisatoren versetzt werden.

Dieses Klebematerial kann teilweise oder vollständig auf der Membran oder direkt auf der Matrix aus Blockmischpolymerisat und Alkenhomopolymerisat aufgetragen sein. Bei vollständiger Bedeckung der Matrix mit Klebschicht kann diese neben ihrer eigentlichen Funktion als Haftmittel auf der Haut ausserdem als permeable Membran wirken. Gewünschte Membraneigenschaften, z.B. Steuerung der Diffusionsgeschwindigkeit des Penetriermittels, lassen sich durch Variation der Stärke und der Zusammensetzung der Klebschicht erzielen. Das Klebematerial kann ausserdem die gesamte oder vorzugsweise eine anteilige Menge des zu applizierenden Wirkstoffs oder der Wirkstoffkombination enthalten. Mit der im Klebematerial enthaltenen Wirkstoffmenge lässt sich insbesondere eine anfängliche stossweise Zufuhr erzielen, bevor die vom therapeutischen System gesteuerte kontinuierliche Zufuhr auf dem gewünschten therapeutischen Niveau einsetzt.

Die Membran oder die Matrix kann ausserdem teilweise und/oder diskontinuierlich mit Klebematerial bedeckt sein. Dabei ist eine randförmige Bedeckung möglich, z.B. ringförmige umlaufende Bedeckung. Die Membran kann auch musterförmig bedeckt sein, z.B. rautenförmig. Die Membran kann am äusseren Rand durch ein kontinuierliches Band von Klebematerial, z.B. ringförmig, und auf der Innenfläche mit diskontinuierlichen Bändern, so dass sich ein rhombisches Muster ergibt, bedeckt sein.

Die im erfindungsgemässen Pflaster enthaltene Reservoirschicht enthält eine Kombination aus Eucalyptol mit einem Gehalt von mindestens 70 % 1,8-Cineol und N-Methyl-2-pyrrolidon als ein die Hautpermeabilität von systemischen Wirkstoffen förderndes Mittel (Penetriermittel), welches die Permeabilität der Wirkstoffe durch die Haut erhöht, so dass eine grössere Menge an Wirkstoffen bezogen auf die Applikationsfläche und Zeiteinheit von der Haut absorbiert wird. Dieses Penetriermittel kann ausserdem den Durchgang der Wirkstoffe durch die Matrix und gegebenenfalls die Membranschicht beschleunigen. Insbesondere wird bei Verwendung dieses Penetriermittels die zur Einhaltung des therapeutischen Niveaus notwendige Dosismenge an Wirkstoffen pro Zeiteinheit durch die Haut appliziert. Das Penetriermittel kann mit weiteren pharmazeutisch annehmbaren Hilfsstoffen vermischt sein.

Der Ausdruck Eucalyptol umfasst Zusammensetzungen mit einem Gehalt von ca. 70-100 % 1,8-Cineol. Es werden auch Zusammensetzungen mit einem Gehalt von ca. 70-95 % 1,8-Cineol in diversen Pharmakopöen (U.S.P. oder Eur. Pharm.) als Eukalyptusöle bezeichnet, während man nur für Zusammensetzungen mit einem Gehalt von mehr als 95 % 1,8-Cineol den Ausdruck Eucalyptol verwendet.

Eukalyptusöle sind terpenhaltige ätherische Oele, welche Eucalyptol bzw. 1,8-Cineol als Hauptbestandteil (mehr als 70 %) oder als alleinige Komponente enthalten und aus Blättern, Wurzeln oder Rinde von Eukalyptuspflanzen der Spezies Eucalyptus globulus (Gemeiner Eukalyptusbaum), Eucalyptus maculata, Eucalyptus cladocalyx oder Eucalyptus sideroxylon isolierbar sind. Durch übliche Reinigungsverfahren, z.B. Rektifizierung, kann Eukalyptusöl zu chemisch reinem 1,8-Cineol (Gehalt mehr als 99 %) weiterverarbeitet werden. In der Reservoirschicht b) ist bevorzugt dieses chemisch reine 1,8-Cineol mit dem zusätzlichen Penetrationsmittel N-Methyl-2-pyrrolidon enthalten.

Bei Verwendung des Penetriermittels und Abwesenheit einer Membranschicht wird die Abgabemenge und Abgabegeschwindigkeit (Abgabemenge pro Zeiteinheit) des in der Reservoirschicht b) enthaltenen Wirkstoffs bzw. einer dort vorhandenen Wirkstoffkombination aus der aus einer Membranschicht bestehenden Reservoirschicht b) an die Haut und die entsprechende Aufnahme (bzw. Aufnahmegeschwindigkeit) durch die Haut beträchtlich gesteigert. So werden in einem Zeitraum bis zu 24 Stunden nach Anbringen des Systems weitgehend konstante Mengen des zu applizierenden Wirkstoffs an die Haut abgegeben. Neben dem Wirkstoff selbst bzw. der Wirkstoffkombination werden ausreichende Mengen der im System enthaltenen Penetriermittel abgegeben, so dass eine Verstärkung der Hautpermeabilität des Wirkstoffs erfolgt und die Aufnahme ausreichender Wirkstoffmengen gewährleistet ist.

Vor allem ist die Kombination aus 5-9,5 Gewichtsteilen chemisch reinem 1,8-Cineol und entsprechend 5-0,5 Gewichtsteilen (bezogen auf 10 Gewichtsteile) N-Methyl-2-pyrrolidon bevorzugt. Solche Kombinationen haben gegenüber den Einzelkomponenten N-Methyl-2-pyrrolidon und 1,8-Cineol den Vorteil, dass diese sich mit dem polymeren Material der Reservoirschicht b) zu homogenen und abgabefähigen Matrixsystemen mit ausreichender Klebeeigenschaft verarbeiten lassen.

Besonders bevorzugt ist die Kombination von ca. 9 Gewichtsteilen chemisch reinem 1,8-Cineol und 1 Gewichtsteil N-Methyl-2-pyrrolidon.

Die zur Erzielung eines therapeutischen Effekts im therapeutischen System enthaltene Wirkstoffmenge ist von vielen Faktoren abhängig: u.a. von der erforderlichen Mindestdosismenge, der Durchlässigkeit der Matrix und gegebenenfalls des Membranmaterials mit dem Klebematerial sowie der Zeitdauer, in der das Pflaster auf der Haut oder den Schleimhäuten befestigt ist. Da sich die Wirkstoffabgabe über einen längeren Zeitraum als einen Tag erstrecken soll, besteht eigentlich keine Obergrenze hinsichtlich der maximalen im Pflaster enthaltenen Wirkstoffmenge. Die Mindestwirkstoffmenge wird vom Erfordernis festgelegt, dass ausreichende Wirkstoffmengen im Pflaster enthalten sein müssen, um die erwünschte Abgabemenge über den vorgesehenen Zeitraum aufrechtzuerhalten.

Dabei lassen sich sämtliche permeationsfähigen Arzneimittel verwenden, welche von der mit dem Pflaster versehenen Hautfläche absorbiert werden. Solche Arzneimittel sind beispielsweise antibakterielle Wirkstoffe wie Penicilline, Tetracycline, Oxytetracycline, Chlortetracycline, Chloramphenicol oder Sulfonamide, Sedativa und/oder Hypnotika wie Pentabarbital-Natrium, Phenobarbital, Secobarbital-Natrium, Codein, Alpha-Bromisovalerylharnstoff, Carbromal oder Natriumphenobarbital, Psychostimulanzien wie 3-(2-Aminopropyl)-indolacetat oder 3-(2-Aminobutyl)indolacetat, Antihypertensiva wie Reserpin, Beruhigungsmittel wie Chlorpromazinhydrochlorid oder Thiopropazathydrochlorid, Hormone wie Adrenocorticosteroide, z.B. 6$\alpha$-Methylprednisolen, androgene Steroide, z.B. Methyltestosteron und Fluoxymesteron, östrogene Steroide, z.B. Oestron, 17$\beta$-Oestradiol und Aethinylöstradiol, Progesteron oder Norethindron, Kombinationen aus Oestrogenen mit synthetischen Gestagenen, z.B. 17$\beta$-Oestradiol mit Norethisteron-17-acetat, Antipyretika wie Acetylsalicylsäure, Morphin und andere Analgetika auf Morphinbasis, gefässerweiternde Mittel, z.B. Nitroglycerin oder Isosorbiddinitrat, Herzglycoside wie Digitoxin oder Ouabin, Beta-Blocker wie Propranolol, Oxprenolol oder Metoprolol, Anticholinergika wie Atropin, Methscopolaminbromid, Scopolamin, Hyoscyamin oder Methscopolamin kombiniert mit Phenobarbital, Antimalariamittel wie 4-Aminochinoline, 9-Aminochinoline oder Pyrimethamin, Entwöhnungsmittel zur Beseitigung von Suchtgefahren, z.B. Nicotin zur Raucherentwöhnung, sowie Broncholytika wie Formoterol.

Besonders bevorzugt sind in der Reservoirschicht b) Nitroglycerin, Scopolamin, Formoterol, 17$\beta$-Oestradiol, Progesteron oder Kombinationen von 17$\beta$-Oestradiol mit Norethisteron-17-acetat als permeationsfähige Wirkstoffe oder Kombinationen vorhanden.

Die genannten Wirkstoffe können in der Reservoirschicht b) in freier Form, z.B. als Säuren oder als Basen, oder als pharmazeutisch annehmbares Salz, z.B. als Chlorid, Bromid, Acetat, Fumarat, Maleat, Succinat, Lactat etc., vorhanden sein.

Die Reservoirschicht b) kann gegebenenfalls weitere Hilfsstoffe enthalten. Geeignet sind Hilfsstoffe wie Wasser, isotonische wässrige Kochsalzlösung, Dextrose in Wasser oder Kochsalzlösung, flüssige Glyceryltriester mit niedermolekularen Fettsäuren, Niederalkanole, natürliche Oele wie Maisöl, Erdnussöl, Sesamöl, Castoröl sowie deren Kondensationsprodukte mit Aethylenoxid und ähnliche, Kohlenwasserstoffe wie Mineralöl pharmazeutischer Qualität, Silicone, Emulgatoren wie Mono- oder Diglyceride von Fettsäuren, Phosphatidsäurederivate wie Lecithin oder Kephalin, Polyalkylenglycole wie Polyäthylenglycol, wässrige Phasen versetzt mit einem Quellmittel wie Natriumcarboxymethylcellulose, Natriumalginat, Polyvinylpolypyrrolidon etc., denen noch Dispersionsmittel oder Emulgatoren wie Lecithin zugesetzt sein können, und ähnliche. Zu diesen Hilfsstoffen können ferner weitere Zusätze wie Konservierungsmittel, Stabilisatoren, Benetzungsmittel etc. zugesetzt werden.

Die von der Reservoirschicht b) abziehbare Schutzfolie c) wird vor der Applikation entfernt. Sie besteht aus Materialien, die für die Bestandteile der Reservoirschicht b) undurchlässig sind. Dabei lassen sich dieselben Materialien, die zur Herstellung der Deckschicht a) dienen können, sowie Metallfolien, z.B. dünne Aluminiumfolie, verwenden. Organische Polymere werden durch geeignete Oberflächenbehandlung, z.B. Siliconbehandlung, von der Haftklebeschicht b) abziehbar gemacht.

Das mehrschichtige Pflaster gemäss vorliegender Erfindung hat vorzugsweise folgende Bestandteile:

a) eine für die Bestandteile der Reservoirschicht b) undurchlässige Deckschicht,

b) eine Wirkstoff-abgabefähige Reservoirschicht, welche aus gegebenenfalls vernetzten Butadien-Styrol-Blockcopolymeren oder Aethylen-Butadien-Styrol-Blockpolymeren vermischt mit Polyisobutylen mit einem Molekulargewichtsbereich von ca. $1{,}0 \times 10^3$ bis $5{,}0 \times 10^4$ besteht und mindestens einen hautpermeationsfähigen Wirkstoff, mindestens ein die Hautpermeabilität von Wirkstoffen förderndes Mittel und gegebenenfalls weitere pharmazeutische Hilfsstoffe enthält, und

c) eine von der Reservoirschicht b) abziehbare Schutzfolie.

Das mehrschichtige Pflaster gemäss vorliegender Erfindung wird hergestellt, indem man auf der abziehbaren Schutzfolie c) die Reservoirschicht mit ihren Bestandteilen und darauf die Deckschicht a) aufträgt oder geänderter Reihenfolge verfährt und auf der Deckschicht a) die Reservoirschicht b) und darauf die Schutzfolie c) aufträgt und nach Durchführung einer der beiden Verfahrensvarianten die verschiedenen Schichten miteinander verbindet und das Pflaster in die gewünschte Form bringt. Dabei wird das Pflaster aus der Vorlage ausgestanzt. Gegebenenfalls wird die Reservoirschicht b) unter Verwendung von zusätzlichem Klebstoff mit der Deckschicht a) verbunden. Ebenso können die verschiedenen Schichten heiss verschweisst werden.

Die Bestandteile der Reservoirschicht b) werden vor der Herstellung des mehrschichtigen Pflasters vermischt, indem man z.B. das Hautpenetriermittel Eucalyptol und N-Methyl-2-pyrrolidon mit dem Blockmischpolymerisat, z.B. einem Copolymeren oder Terpolymeren aus der Gruppe CARIFLEX oder KRATON, und dem Alkenhomopolymeren, z.B. einem Polyisobutylen aus der Gruppe OPPANOL, vor allem durch Kneten zu einer homogenen Masse verarbeitet, vorzugsweise bei Temperaturen bis zu 100 °C. Die viskose Masse lässt sich nach Auftragen auf die Deckschicht a), z.B. siliconisierte Polyesterfolie, oder auf die Folie c) (Release liner) auswalzen. Die Vernetzung der Polymeren kann durch Bestrahlen der Beschichtung mit energiereichem Licht, z.B. durch UV-Licht, z.B. mit einer Quecksilberlampe als Quelle, gegebenenfalls unter Verwendung eines geeigneten Fotoinitiators wie 2-Hydroxy-2-methyl-1-phenylpropan-1-on (Darocur® Merck) oder 2,2-Dimethoxy-1,2-Diphenylethan-1-on (Irgacure® 651, Ciba-Geigy) erfolgen. Auf das vernetzte polymere Material lässt sich dann der Wirkstoff, gegebenenfalls gelöst in etwas Penetriermittel, auftragen. Alternativ kann der Wirkstoff mit dem polymeren Material vermischt werden. Zur weiteren Sättigung der Haftklebeschicht kann man diese mit weiterem Penetriermittel oder Wirkstoffhaltiger Lösung des Penetriermittels mehrere Stunden bis Tage quellen lassen.

Die Herstellungsverfahren und Anwendungen sind in den U.S. Patentschriften Nr. 3,598,122, 3,598,123, 3,797,494 und 4,060,084 beschrieben, vorzugsweise in der DE-A-26 04 718 und DE-A-32 05 258 bzw. in den U.S. Patentschriften 4,031,894 und 4,262,003 oder in der Publikation Schweiz. Rundschau Med. (Praxis) 74, Nr. 11, 257-260 (1985) für die Herstellung von Matrix- oder Monolith-Systemen, wobei die erfindungsgemässe Anwendung nicht auf die in diesen Publikationen beschriebenen transdermalen therapeutischen Systeme beschränkt ist.

Beispiel 1:

1a) 52 g einer Mischung von 1,8-Cineol mit N-Methyl-2-pyrrolidon (9 Gewichtsteile 1,8-Cineol und 1 Gewichtsteil N-Methyl-2-pyrrolidon) werden mit 33,6 g Styrol-Butadien-Blockcopolymer vom Typ CARIFLEX TR 1107 und 14,4 g Polyisobutylen mit einem durchschnittlichen Molekulargewicht von $4 \times 10^4$ vom Typ OPPANOL B-10 zu einer homogenen Masse verarbeitet.

1b) Ein 1 mm starker Metallrahmen mit rechteckiger Oeffnung (3 x 8 cm) wird auf eine Abdeckfolie von ca. 0,1 mm Stärke aus aluminisiertem Polyester (PE 3M Co. USA) aufgelegt. In die Oeffnung des Metallrahmens werden 3,2 g der gemäss 1a) hergestellten Formulierung gebracht und mit einer Abziehfolie aus siliconisiertem Polyester (DuPont) abgedckt. Man verpresst mit Hilfe einer heizbaren Laborpresse vom Typ Jauch bei einer Arbeitstemperatur von ca. 70°C zu einem Verbundlaminat, wobei man die Schichten des Laminats ca. 30 sec. lang bei einem Druck von ca. 6 bar miteinander verbindet. Das plattenförmige Laminat wird aus dem Rahmen genommen, von der Abziehfolie befreit und 120 sec. lang mit UV-Licht bestrahlt (Minicure Marck 2, Primarc Ltd.)

Man trägt ca. 2 Gew.-% Progesteron (bezogen auf die Monolithmasse) auf, indem man das Progesteron in etwas 1,8-Cineol und N-methylpyrrolidon löst und die Lösung auf die Platte sprüht. Man deckt das mit Wirkstoff beladene Laminat wieder mit Abziehfolie durch Andrücken ab und stanzt transdermale Systeme in Form von Pflastern geeigneter Grösse, z.B. von ca. 2,6 cm Durchmesser, aus dem Laminat aus.

c) Zur Herstellung von Pflastern mit besonders dünner Reservoirschicht (weniger als 50 $\mu$m Stärke) werden die Bestandteile der Haftklebeschicht in Heptan gelöst und die Lösung auf die Deckschicht aus aluminisiertem Polyester so aufgegossen, dass nach Verdampfen des Lösungsmittels eine Monolithschicht der gewünschten Stärke resultiert. Die übrigen Verfahrensschritte sind analog.

Beispiel 2:

2a) Eine Mischung von 39,9 Gewichtsteilen Styrol-Butadien-Blockcopolymer CARIFLEX TR 1107 und 26,6 Gewichtsteilen Polyisobutylen OPPANOL B-10 wird mit einer Lösung aus 5 Gewichtsteilen 17$\beta$-Oestradiol in 2,85 Gewichtsteilen N-Methyl-2-pyrrolidon und 26,65 Gewichtsteilen 1,8-Cineol zu einer homogenen Masse vermischt.

2b) Analog Beispiel 1b), jedoch unbelichtet, werden 3,2 g dieser Formulierung auf eine Abdeckfolie gegeben, mit Abziehfolie versehen, verpresst und zu transdermalen Systemen in Form von Pflastern mit 2,6 cm Durchmesser ausgestanzt. Abmessungen und Abgabeeigenschaften dieser Systeme sind in der Tabelle angegeben.

2c) Die Monolithmasse gemäss Beispiel 2a) wird ausserdem noch mit zwei Gewichtsteilen 2-Hydroxy-2-methyl-1-phenylpropan-1-on (Darocur® 1173, Merck) versetzt. In Abwandlung von Beispiel 1b) werden 3,2 g dieser Formulierung auf eine erste Abziehfolie - statt auf eine Abdeckfolie - gegeben und mit einer zweiten Abziehfolie abgedeckt. Man verpresst zu einem Laminat und entfernt auf einer Seite die erste Abziehfolie. Man bestrahlt die nicht beschichtete Seite 42 sec. lang mit UV-Licht und deckt diese Seite mit Abdeckfolie ab. Man verpresst nochmals und entfernt die zweite Abziehfolie. Man bestrahlt wiederum 42 sec. lang die andere, unbeschichtete Seite mit UV-Licht, versieht diese offene Seite der Haftklebeschicht mit Abziehfolie durch Andrücken und stanzt die Systeme in Form von Pflastern aus. Abmessungen und Abgabeeigenschaften dieser Systeme sind in der Tabelle angegeben.

2d) Die Formulierung von Beispiel 2a) wird mit 2-Hydroxy-2-methyl-1-phenylpropan-1-on (2 Gewichtsteile) versetzt und auf eine erste Abziehfolie gegeben. Man schliesst mit einer zweiten Abziehfolie ab, verpresst zu einem Laminat und stanzt in Form von Pflastern aus. Nach dem Entfernen der ersten Abziehfolie auf einer Seite wird 42 sec. lang mit UV-Licht bestrahlt. Man deckt die belichtete Seite wieder mit Abziehfolie ab, entfernt die Abziehfolie auf der unbelichteten Seite und bestrahlt auch diese Seite 42 sec. lang mit UV-Licht. Nach dem erneuten Entfernen beider Abziehfolien wird das Monolithsystem 96 Stunden lang in einer 5%igen (Gew.) 17$\beta$-Oestradiol Lösung in N-Methyl-2-pyrrolidon und 1,8-Cineol (1:9 w/w) gelagert. Die gequollene Matrixformulierung wird anschliessend mit Abdeckfolie und Abziehfolie beschichtet und verpresst. Abmessungen und Abgabeeigenschaften dieser Systeme sind in der Tabelle angegeben.

2e) Analog Beispiel 2d) werden transdermale therapeutische Systeme mit 17$\beta$-Oestradiol hergestellt, wobei beide Seiten der Haftklebeschicht 67 sec. mit UV-Licht bestrahlt werden. Abmessungen und Abgabeeigenschaften dieser Systeme sind in der Tabelle angegeben.

2f) Die Permeationseigenschaften der Systeme in vitro gemäss Beispiel 2b)-2e) durch Messung der Kumulativen Permeation des Wirkstoffs 17$\beta$-Oestradiol durch Schweineepidermis wird wie folgt bestimmt: Vom betreffenden System wird die Abziehfolie entfernt und die freigelegte Fläche der Haftklebeschicht auf die Schweineepidermis durch Andrücken befestigt. Die Schweineepidermis mit Pflaster wird in einer Diffusionszelle nach T.J. Franz (J.Invest.Dermatol. 64, 190 (1975)), montiert. Als Akzeptor wird Bovine-Serum-Albumin-Lösung benutzt. Der Gehalt an 17$\beta$-Oestradiol in Akzeptorflüssigkeit wird mittels HPLC bestimmt. Die Messwerte sind in der Tabelle wiedergegeben.

Tabelle Abmessungen und Permeationseigenschaften von Monolithsystemen in vitro.

| Beispiel | Abmessungen Monolithsystem | | | Kumulative Permeation | |
|---|---|---|---|---|---|
| Nr. | Durchmesser [mm] | Stärke [mm] | Gewicht [mg] | nach 24 Stunden [µg/cm²] | nach 48 Stunden[µg/cm²] |
| 2b | 26 | 1 | 470 | 4,4 | 6,9 |
| 2c | 26 | 1 | 470 | 9,8 | 15,3 |
| 2d | 22/49 1) | 1/3 1) | 400/5600 1) | 67,4 | 116,2 |
| 2e | 22/48 1) | 1/2,5 1) | 400/4400 1) | 39,9 | 60,2 |

1) nach Quellung

Beispiel 3: Analog Beispiel 2d) werden Monolithsysteme hergestellt, welche durch einen Gehalt von ca. 0,2-0,5 % Formoterol in der Haftklebeschicht gekennzeichnet sind.

**Patentansprüche**
**Patentansprüche für folgende Vertragsstaaten : AT, BE, CH, DE, FR, GB, IT, LI, LU, NL, SE**

1.  Transdermales therapeutisches System in Form eines mehrschichtigen Pflasters mit folgenden Bestandteilen:
    a) einer für die Bestandteile der Reservoirschicht b) undurchlässigen Deckschicht;
    b) einer Wirkstoff-abgabefahigen Reservoirschicht, welche aus gegebenenfalls vennetztem Blockmischcopolymerisat auf der Basis von Styrol, Alkadienen und gegebenenfalls Alkenen vermischt mit klebefähigen Polymeren besteht und gegebenenfalls mit einer zusätzlichen klebefähigen Membranschicht versehen ist, und mindestens einen hautpermeationsfähigen Wirkstoff, eine Kombination aus Eucalyptol mit einem Gehalt von mindestens 70 % 1,8-Cineol und N-Methyl-2-pyrrolidon als ein die Hautpermeabilität von Wirkstoffen förderndes Mittel und gegebenenfalls weitere pharmazeutische Hilfsstoffe enthält, und
    c) einer von der Reservoirschicht b) abziehbaren Schutzfolie.

2.  Transdermales therapeutisches System gemäß Anspruch 1, dadurch gekennzeichnet, daß die Reservoirschicht b) eine Kombination aus chemisch reinem 1,8-Cineol (Reinheitsgrad höher als 99 %) und N-Methyl-2-pyrrolidon als ein die Hautpermeabilität von Wirkstoffen förderndes Mittel enthält.

3.  Transdermales therapeutisches System gemäß Anspruch 1, dadurch gekennzeichnet, daß die Kombination aus 1,8-Cineol und N-Methyl-2-pyrrolidon ca. 5-9,5 Gewichtsteile 1,8-Cineol und entsprechend ca. 5-0,5 Gewichtsteile (bezogen auf 10) N-Methyl-2-pyrrolidon enthält.

4.  Transdermales therapeutisches System gemäß Anspruch 3, dadurch gekennzeichnet, daß die Kombination aus 1,8-Cineol und N-Methyl-2-pyrrolidon ca. 9 Gewichtsteile 1,8-Cineol und 1 Gewichtsteil N-Methyl-2-pyrrolidon enthält.

5.  Transdermales therapeutisches System gemäß einem der Ansprüche 1-4, dadurch gekennzeichnet, daß die Reservoirschicht b) Nitroglycerin, Scopolamin, Formoterol, 17$\beta$-Oestradiol oder Progesteron oder Kombinationen von 17$\beta$-Oestradiol mit Norethisteron- 17-acetat als permeationsfähige Wirkstoffe enthält.

6.  Transdermales therapeutisches System gemäß einem der Ansprüche 1-5, dadurch gekennzeichnet, daß die Reservoirschicht b) aus Aethylen-Butadien-Styrol-Blockcopolymeren oder Butadien-Styrol-Blockcopolymeren besteht, welche gegebenenfalls durch Einwirken von energiereicher Strahlung ganz oder teilweise vernetzt und welche mit Polyisobutylen mit einem durchschnittlichen Molekulargewicht von $1,0 \cdot 10^3$ bis $1,0 \cdot 10^4$ vermischt sind.

7.  Verfahren zur Herstellung eines transdermalen, therapeutischen Systems gemäß Anspruch 1, dadurch gekennzeichnet, daß man auf der abziehbaren Schutzfolie c) die Reservoirschicht b) mit ihren Bestandteilen und darauf die Deckschicht a) aufträgt oder in geänderter Reihenfolge verfährt und auf der Deckschicht a) die Reservoirschicht b) und darauf die Schutzfolie c) aufträgt und nach Durchführung einer der beiden Verfahrensvarianten die Schichten miteinander verbindet und das System in die gewünschte Form bringt.

8.  Pharmazeutisches Pflaster gemäß Anspruch 1 zur transdermalen Anwendung in einem therapeutischen Verfahren am menschlichen oder tierischen Körper.

9.  Verwendung eines Gemisches aus 1,8-Cineol und N-Methyl-2-pyrrolidon zur Herstellung eines transdermalen, therapeutischen Systems gemaß Anspruch 1.

10. Verwendung gemäß Anspruch 9 eines Gemisches aus 9 Gewichtsteilen 1,8-Cineol und 1 Gewichtsteil N-Methyl-2-pyrrolidon zur Herstellung eines transdermalen, therapeutischen Systems gemäß Anspruch 1.

**Patentansprüche für folgende Vertragsstaaten : ES, GR**

1.  Verfahren zur Herstellung eines transdermalen therapeutischen Systems in Form eines mehrschichtigen Pflasters mit folgenden Bestandteilen:
    a) einer für die Bestandteile der Reservoirschicht b) undurchlässigen Deckschicht;
    b) einer Wirkstoff-abgabefähigen Reservoirschicht, welche aus gegebenenfalls vernetztem Blockmischcopolymerisat auf der Basis von Styrol, Alkadienen und gegebenenfalls Alkenen vermischt mit klebefähigen Polymeren besteht und gegebenenfalls mit einer zusätzlichen klebefähigen Membranschicht versehen ist, und mindestens einen hautpermeationsfähigen Wirkstoff, eine Kombination aus Eucalyptol mit einem Gehalt von mindestens 70 % 1,8-Cineol und N-Methyl-2-pyrrolidon als ein die Hautpermeabilität von Wirkstoffen förderndes Mittel und gegebenenfalls weitere pharmazeutische Hilfsstoffe enthält, und

c) einer von der Reservoirschicht b) abziehbaren Schutzfolie, dadurch gekennzeichnet, daß man auf der abziehbaren Schutzfolie c) die Reservoirschicht b) mit ihren Bestandteilen und darauf die Deckschicht a) aufträgt oder in geänderter Reihenfolge verfährt und auf der Deckschicht a) die Reservoirschicht b) und darauf die Schutzfolie c) aufträgt und nach Durchführung einer der beiden Verfahrensvarianten die Schichten miteinander verbindet und das System in die gewünschte Form bringt.

2. Verfahren zur Herstellung eines transdermalen therapeutischen Systems gemäß Anspruch 1, dadurch gekennzeichnet, daß die Reservoirschicht b) eine Kombination aus chemisch reinem 1,8-Cineol (Reinheitsgrad höher als 99 %) und N-Methyl-2-pyrrolidon als ein die Hautpermeabilität von Wirkstoffen förderndes Mittel enthält.

3. Verfahren zur Herstellung eines transdermalen therapeutischen Systems gemäß Anspruch 1, dadurch gekennzeichnet, daß die Kombination aus 1,8-Cineol und N-Methyl-2-pyrrolidon ca. 5-9,5 Gewichtsteile 1,8-Cineol und entsprechend ca. 5-0,5 Gewichtsteile (bezogen auf 10) N-Methyl-2-pyrrolidon enthält.

4. Verfahren zur Herstellung eines transdermalen therapeutischen Systems gemäß Anspruch 3, dadurch gekennzeichnet, daß die Kombination aus 1,8-Cineol und N-Methyl-2-pyrrolidon ca. 9 Gewichtsteile 1,8-Cineol und 1 Gewichtsteil N-Methyl-2-pyrrolidon enthält.

5. Verfahren zur Herstellung eines transdermalen therapeutischen Systems gemaß einem der Ansprüche 1-4, dadurch gekennzeichnet, daß die Reservoirschicht b) Nitroglycerin, Scopolamin, Formoterol, 17$\beta$-Oestradiol oder Progesteron oder Kombinationen von 17$\beta$-Oestradiol mit Norethisteron-17-acetat als permeationsfähige Wirkstoffe enthält.

6. Verfahren zur Herstellung eines transdermalen therapeutischen Systems gemäß einem der Ansprüche 1-5, dadurch gekennzeichnet, daß die Reservoirschicht b) aus AethylenButadien-Styrol-Blockcopolymeren oder Butadien-Styrol-Blockcopolymeren besteht, welche gegebenenfalls durch Einwirken von energiereicher Strahlung ganz oder teilweise vernetzt und welche mit Polyisobutylen mit einem durchschnittlichen Molekulargewicht von $1,0 \cdot 10^3$ bis $1,0 \cdot 10^4$ vermischt sind.

## Claims

**Claims for the following Contracting States : AT, BE, CH, DE, FR, GB, IT, LI, LU, NL, SE**

1. A transdermal therapeutic system in the form of a multilayer plaster with the following constituents:

a) a covering layer which is impermeable to the constituents of the reservoir layer b);

b) a reservoir layer which is able to deliver active substance and which consists of optionally crosslinked block copolymer based on styrene, alkadienes and optionally alkenes mixed with alkene or alkadiene homopolymer optionally provided with an adhesive membrane layer and contains at least one active substance capable of skin permeation, a combination of eucalyptol with a content of at least 70 % 1,8-cineole and N-methyl-2-pyrrolidone as an agent promoting the skin permeability of active substances and optionally further pharmaceutical auxiliaries, and

c) a protective film which can be pulled off the reservoir layer b).

2. A transdermal therapeutic system according to claim 1, characterized in that the reservoir layer b) contains a combination of chemically pure 1,8-cineole (purity greater than 99 %) and N-methyl-2-pyrrolidone as an agent promoting the skin permeability of active substances.

3. A transdermal therapeutic system according to claim 1, characterized in that the combination of 1,8-cineole and N-methyl-2-pyrrolidone contains about 5-9.5 parts by weight of 1,8-cineole and, correspondingly, about 5-0.5 parts by weight (based on 10) of N-methyl-2-pyrrolidone.

4. A transdermal therapeutic system according to claim 3, characterized in that the combination of 1,8-cineole and N-methyl-2-pyrrolidone contains about 9 parts by weight of 1,8-cineole and 1 part by weight of N-methyl-2-pyrrolidone.

5. A transdermal therapeutic system according to one of claims 1-4, characterized in that the reservoir layer b) contains nitroglycerine, scopolamine, formoterol, 17$\beta$-oestradiol or progesterone or combinations of 17$\beta$-oestradiol with norethisterone 17-acetate as active substances which are able to permeate.

6.  A transdermal therapeutic system according to one of claims 1-5, characterized in that the reservoir layer b) consists of ethylene/butadiene/styrene block terpolymers or butadiene/styrene block copolymers, which may be wholly or partially crosslinked by exposure to ultraviolet radiation and which are mixed with polyisobutylene with an average molecular weight of $1.0 \times 10^3$ to $1 \times 10^4$.

7.  A process for the production of a transdermal therapeutic system according to claim 1, characterized in that applied to the protective layer c) which can be pulled off are the reservoir layer b) with its constituents and, thereto, the covering layer a), or the sequence is altered and applied to the covering layer a) are the reservoir layer b) and, thereto, the protective film c), and after one of the two process variants has been carried out, the layers are bonded together and the system is brought to the desired shape.

8.  A pharmaceutical plaster according to claim 1, for transdermal use in a therapeutic procedure on the human or animal body.

9.  The use of a mixture of 1,8-cineole and N-methyl-2-pyrrolidone for the production of a transdermal therapeutic system according to claim 1.

10. The use according to claim 9 of a mixture of 9 parts by weight of 1,8-cineole and 1 part by weight of N-methyl-2-pyrrolidone for the production of a transdermal therapeutic system according to claim 1.

**Claims for the following Contracting States : ES, GR**

1.  A process for the production of a transdermal therapeutic system in the form of a multilayer plaster with the following constituents:
    a) a covering layer which is impermeable to the constituents of the reservoir layer b);
    b) a reservoir layer which is able to deliver active substance and which consists of optionally crosslinked block copolymer based on styrene, alkadienes and optionally alkenes mixed with alkene or alkadiene homopolymer optionally provided with an adhesive membrane layer and contains at least one active substance capable of skin permeation, a combination of eucalyptol with a content of at least 70 % 1,8-cineole and N-methyl-2-pyrrolidone as at least one agent promoting the skin permeability of active substances and optionally further pharmaceutical auxiliaries, and
    c) a protective film which can be pulled off the reservoir layer b), characterized in that applied to the protective layer c) which can be pulled off are the reservoir layer b) with its constituents and, thereto, the covering layer a), or the sequence is altered and applied to the covering layer a) are the reservoir layer b) and, thereto, the protective film c), and after one of the two process variants has been carried out, the layers are bounded together and the system is brought to the desired shape.

2.  A process for the production of a transdermal therapeutic system according to claim 1, characterized in that the reservoir layer b) contains a combination of chemically pure 1,8-cineole (purity greater than 99 %) and N-methyl-2-pyrrolidone as an agent promoting the skin permeability of active substances.

3.  A process for the production of a transdermal therapeutic system according to claim 1, characterized in that the combination of 1,8-cineole and N-methyl-2-pyrrolidone contains about 5-9.5 parts by weight of 1,8-cineole and, correspondingly, about 5-0.5 parts by weight (based on 10) of N-methyl-2-pyrrolidone.

4.  A process for the production of a transdermal therapeutic system according to claim 3, characterized in that the combination of 1,8-cineole and N-methyl-2-pyrrolidone contains about 9 parts by weight of 1,8-cineole and 1 part by weight of N-methyl-2-pyrrolidone.

5.  A process for the production of a transdermal therapeutic system according to one of claims 1-4, characterized in that the reservoir layer b) contains nitroglycerine, scopolamine, formoterol, 17$\beta$-oestradiol or progesterone or combinations of 17$\beta$-oestradiol with norethisterone 17-acetate as active substances which are able to permeate.

6.  A process for the production of a transdermal therapeutic system according to one of claims 1-5, characterized in that the reservoir layer b) consists of ethylene/butadiene/styrene block terpolymers or butadiene/styrene block copolymers, which may be wholly or partially crosslinked by exposure to ultraviolet radiation and which are mixed with polyisobutylene with an average molecular weight of $1.0 \times 10^3$ to 1

x $10^4$ .

**Revendications**
**Revendications pour les Etats contractants suivants : AT, BE, CH, DE, FR, GB, IT, LI, LU, NL, SE**

1. Système thérapeutique trans-dermique sous forme d'emplâtres à plusieurs couches, avec les composants suivants :
   a) une couche de couverture imperméable pour les constituants de la couche-réservoir b),
   b) une couche-réservoir capable de libérer une substance active, qui consiste en un copolymère séquencé, éventuellement réticulé, à base de styrène, d'alcadiènes et le cas échéant d'alcènes, en mélange avec des polymères adhésifs, et qui porte le cas échéant une autre couche de membrane adhésive, et contient au moins une substance active apte à la perméation au travers de la peau, une combinaison d'eucalyptol à une teneur d'au moins 70% en 1,8-cinéol et de N-méthyl-2-pyrrolidone en tant qu'agent accroissant la perméabilité de la peau pour les substances actives, et le cas échéant d'autres produits auxiliaires pharmaceutiques, et
   c) une feuille de protection détachable de la couche-réservoir b).

2. Système thérapeutique trans-dermique selon revendication 1, caractérisé en ce que la coucheréservoir b) contient une combinaison de 1,8-cinéol chimiquement pur (pureté supérieure à 99%) et de N-méthyl-2-pyrrolidone en tant qu'agent accroissant la perméabilité de la peau pour les substances actives.

3. Système thérapeutique trans-dermique selon revendication 1, caractérisé en ce que la combinaison de 1,8-cinéol et de N-méthyl-2-pyrrolidone contient environ 5 à 9,5 parties en poids de 1,8-cinéol et, en correspondance environ 5 à 0,5 parties en poids (pour 10) de N-méthyl-2-pyrrolidone.

4. Système thérapeutique trans-dermique selon revendication 3, caractérisé en ce que la combinaison de 1,8-cinéol et de N-méthyl-2-pyrrolidone contient environ 9 parties en poids de 1,8-cinéol et 1 partie en poids de N-méthyl-2-pyrrolidone.

5. Système thérapeutique trans-dermique selon une des revendications 1 à 4, caractérisé en ce que la couche-réservoir b) contient de la nitroglycérine, de la scopolamine, du formotérol, du 17 bêta-oestradiol ou de la progestérone ou une combinaison de 17 bêta-oestradiol et de 17-acétate de noréthistérone, en tant que substance active apte à la perméation.

6. Système thérapeutique trans-dermique selon une des revendications 1 à 5, caractérisé en ce que la couche-réservoir b) consiste en copolymères séquencés éthylène-butadiène-styrène ou copolymères séquencés butadiène-styrène, éventuellement réticulés en totalité ou en partie par action de rayonnements à haute énergie et qui sont mélangés avec un polyisobutylène au poids moléculaire moyen de 1,0 x $10^3$ à 1,0 x $10^4$ .

7. Procédé de préparation d'un système thérapeutique trans-dermique selon revendication 1, caractérisé en ce que, sur la couche de protection détachable c) on applique la couche-réservoir b) avec ses constituants et, par dessus, la couche de couverture a), ou bien, dans un ordre différent, on applique sur la couche de couverture a) la couche-réservoir b) et, par dessus, la couche protectrice c) et après cet assemblage, dans un ordre ou dans l'autre, on fixe les couches entre elles et on met le système sous la forme voulue.

8. Emplâtre pharmaceutique selon revendication 1, pour l'administration trans-dermique dans un procédé thérapeutique sur l'organisme humain ou animal.

9. Utilisation d'un mélange de 1,8-cinéol et de N-méthyl-2-pyrrolidone pour la préparation d'un système thérapeutique trans-dermique selon revendication 1.

10. Utilisation selon revendication 9, d'un mélange de 9 parties en poids de 1,8-cinéol et 1 partie en poids de N-méthyl-2-pyrrolidone pour la préparation d'un système thérapeutique trans-dermique selon revendication 1.

**Revendications pour les Etats contractants suivants : ES, GR**

1. Procédé de préparation d'un système thérapeutique trans-dermique sous forme d'un emplâtre à plusieurs couches avec les constituants suivants :
   a) une couche de couverture imperméable pour les constituants de la couche-réservoir b),
   b) une couche-réservoir capable de libérer la substance active, qui consiste en un copolymère séquencé éventuellement réticulé à base de styrène, d'alcadiènes et le cas échéant d'alcènes, en mélange avec des polymères adhésifs, et porte le cas échéant une autre couche de membrane adhésive, et qui contient au moins une substance active apte à la perméation au travers de la peau, une combinaison d'eucalyptol à une teneur d'au moins 70% de 1,8-cinéol et de N-méthyl-2-pyrrolidone en tant qu'agent accroissant la perméabilité de la peau pour les substances actives et le cas échéant d'autres produits auxiliaires pharmaceutiques, et c) une feuille de protection détachable de la couche-réservoir b), ce procédé se caractérisant en ce que, sur la couche de protection détachable c) on applique la couche-réservoir b) avec ses constituants et, par dessus, la couche de couverture a) ou bien, dans un ordre différent, on applique sur la couche de couverture a) la couche-réservoir b) et, par dessus, la feuille de protection c) et, après cet assemblage dans un ordre ou dans l'autre, on fixe les couches entre elles et on met le système à la forme voulue.

2. Procédé de préparation d'un système thérapeutique trans-dermique selon revendication 1, caractérisé en ce que la couche-réservoir b) contient une combinaison de 1,8-cinéol chimiquement pur (pureté supérieure à 99%) et de N-méthyl-2-pyrrolidone en tant qu'agent accroissant la perméabilité de la peau pour les substances actives.

3. Procédé de préparation d'un système thérapeutique trans-dermique selon revendication 1, caractérisé en ce que la combinaison de 1,8-cinéol et de N-méthyl-2-pyrrolidone contient environ 5 à 9,5 parties en poids de 1,8-cinéol et, en correspondance, environ 5 à 0,5 parties en poids (pour 10) de N-méthyl-2-pyrrolidone.

4. Procédé de préparation d'un système thérapeutique trans-dermique selon revendication 3, caractérisé en ce que la combinaison de 1,8-cinéol et de N-méthyl-2-pyrrolidone contient environ 9 parties en poids de 1,8-cinéol et 1 partie en poids de N-méthyl-2-pyrrolidone.

5. Procédé de préparation d'un système thérapeutique trans-dermique selon l'une des revendications 1 à 4, caractérisé en ce que la couche-réservoir b) contient de la nitroglycérine, de la scopolamine, du formotérol, du 17 bêta-oestradiol ou de la progestérone ou une combinaison de 17 bêta-oestradiol et de 17-acétate de noréthistérone en tant que substance active apte à la perméation.

6. Procédé de préparation d'u système thérapeutique trans-dermique selon l'une des revendications 1 à 5, caractérisé en ce que la couche-réservoir b) consiste en copolymères séquencés éthylènebutadiène-styrène ou copolymères séquencés butadiènestyrène éventuellement réticulés en totalité ou en partie par l'action de rayonnements à haute énergie et qui sont mélangés avec du polyisobutylène à un poids moléculaire moyen de $1{,}0 \times 10^3$ à $1{,}0 \times 10^4$.